# EUROPÄISCHE PATENTANMELDUNG

(11) **EP 0 632 024 A1**
(43) Veröffentlichungstag der Anmeldung: **04.01.1995**
(21) Anmeldenummer: 94109146.4
(22) Anmeldetag: 15.06.1994
(51) Int. Cl.: C07D 209/08, C07D 209/18

(54) **Verfahren zur Herstellung von Indolen**

(30) Priorität: 23.06.1993 DE 4320835
(71) Anmelder: MERCK PATENT GmbH, D-64293 Darmstadt (DE)
(72) Erfinder: Hopf, Martin, Dr., D-64846 Gross-Zimmern (DE)

(57) **Zusammenfassung**

Die Erfindung betrifft ein neues Verfahren zur Herstellung von Indolen durch Decarboxylierung entsprechender Indol-2-carbonsäuren, dadurch gekennzeichnet, daß man diese mit Ameisensäure behandelt.

## Beschreibung

Die Erfindung betrifft ein neues Verfahren zur Herstellung von Indolen durch Decarboxylierung entsprechender Indol-2-carbonsäuren.

Indol-2-carbonsäuren wurden üblicherweise decarboxyliert, indem ihre Lösung in einem hochsiedenden tertiären Amin zum Rückfluß erhitzt wird (vgl. z.B. Houben-Weyl/Müller, Methoden der organischen Chemie; 1952, Band 8, Seite 486).

So wurde bisher die 4-(5-Methoxy-3-indolyl) -buttersäure (I), ein Zwischenprodukt für die Synthese von Roxindol, hergestellt durch 24-stündiges Erhitzen einer Lösung von 3-(3-Carboxypropyl)-5-methoxyindol-2-carbonsäure (II) in N,N-Dimethylanilin oder N,N-Diethylanilin auf 190°.

Nachteilig ist dabei die hohe Reaktionstemperatur, die apparative Probleme mit sich bringt und eine Produktion nur in wenigen Anlagen zuläßt, sowie die lange Reaktionsdauer und damit ein hoher Energieverbrauch.

Der Erfindung lag die Aufgabe zugrunde, ein neues Herstellungsverfahren für Indole, insbesondere I, aufzufinden, das die genannten Nachteile nicht oder nur in geringerem Maße aufweist.

Es wurde gefunden, daß man beim Erhitzen einer Lösung von II in Ameisensäure in hoher Ausbeute I erhält. Das ist insofern überraschend, als Indole allgemein als sehr labil gegen Säuren gelten. Dies zeigte sich auch bei Versuchen, Ameisensäure durch andere Säuren wie Essigsäure, verdünnte wässerige Schwefelsäure oder verdünnte wässerige Salzsäure zu ersetzen. Es erfolgte entweder gar keine Umsetzung oder I entstand nur in niedriger Ausbeute unter Bildung von Nebenprodukten.

Das Verfahren läßt sich mit gutem Erfolg auf andere Indol-2-carbonsäuren übertragen.

Gegenstand der Erfindung ist ein Verfahren zur Herstellung von Indolen durch Decarboxylierung entsprechender Indol-2-carbonsäuren, dadurch gekennzeichnet, daß man diese mit Ameisensäure behandelt.

Bei diesem Verfahren verwendet man Ameisensäure, die zweckmäßig etwa 0-50 % Wasser enthalten kann. Die Reaktionstemperaturen liegen zwischen 20 und 107°, vorzugsweise zwischen 80 und 107 °C. Bei den höheren Temperaturen braucht man Reaktionszeiten zwischen etwa 1 und 10, vorzugsweise zwischen 2 und 4 Stunden, bei den niedrigeren Temperaturen entsprechend längere Zeiten.

Bei den nachstehend angegebenen Prozentangaben handelt es sich um Volumensprozente.

### Beispiel 1

Ein Gemisch von 14,2 g II und 120 g 77,4 %iger Ameisensäure wird 3 Stunden gekocht und dann unter vermindertem Druck eingedampft. Man gibt verdünnte Natronlauge hinzu, klärt kurz mit Aktivkohle bei 40° und fällt mit Salzsäure bis pH 1,5. Die ausgefallene I wird abfiltriert, mit Wasser gewaschen und unter vermindertem Druck bei 50° getrocknet. Ausbeute: 10,9 g.

### Beispiel 2

Man arbeitet wie in Beispiel 1 angegeben, aber mit 100 %iger Ameisensäure und benötigt eine Reaktionszeit von 2 Stunden.

### Beispiel 3

Man arbeitet wie in Beispiel 1 angegeben, aber mit 50 %iger Ameisensäure; Reaktionszeit: 5 Stunden.

### Beispiel 4

Man arbeitet wie in Beispiel 1 angegeben, aber bei 80°; Reaktionszeit: 30 Stunden.

### Beispiel 5

Analog Beispiel 1 erhält man Indol aus Indol-2-carbonsäure.

## Patentansprüche

1. Verfahren zur Herstellung von Indolen durch Decarboxylierung entsprechender Indol-2-carbonsäuren, dadurch gekennzeichnet, daß man diese mit Ameisensäure behandelt.
